# EUROPEAN PATENT SPECIFICATION

(11) **EP 4 423 102 B1**
(45) Date of publication and mention of the grant of the patent: **25.02.2026**
(21) Application number: 22761990.5
(22) Date of filing: 04.08.2022
(51) Int. Cl.: C12Q 1/68, C12Q 1/6841, C12Q 1/6834, C12Q 1/6837, C12Q 1/6858, G01N 33/48, C07H 21/00

(54) **METHOD OF MOUNTING A BIOLOGICAL SAMPLE ON A SURFACE**
VERFAHREN ZUR BEFESTIGUNG EINER BIOLOGISCHEN PROBE AUF EINER OBERFLÄCHE
MÉTHODE DE MONTAGE D'UN ÉCHANTILLON BIOLOGIQUE SUR UNE SURFACE

(30) Priority: 25.10.2021 US 202163271392 P
(43) Date of publication of application: 04.09.2024
(73) Proprietor: Resolve BioSciences GmbH, 40789 Monheim am Rhein (DE)
(72) Inventor: KORFHAGE, Christian, 40764 Langenfeld (DE); FABER, Cynthia, 42327 Wuppertal (DE); MEIER, Andreas, 40227 Düsseldorf (DE)
(74) Representative: Roth, Andy Stefan
(86) International application number: PCT/EP2022/071967
(87) International publication number: WO 2023/072448

(56) References cited:
- WO-A1-98/39481
- WO-A2-01/73123
- WO-A2-98/20020
- GB-A- 2 233 654

## Description

### FIELD OF THE DISCLOSURE

The present invention provides a method mounting/sticking a biological sample on a surface by contacting said biological sample on said surface with a reducing agent.

### BACKGROUND

Tissue sections, cells tissue or other biological fluids or materials obtained from clinical specimens or animal experiments frequently are mounted, fixed and stored in a form suitable for examination by microscopy. Mounting is the process by which the specimen is adhered to the microscope slide and protected from physical damage. Generally, in microscopy, a slide of a material or sample to be investigated is prepared by putting said sample or material onto a (bottom) slide and then covering said sample with a (top) cover. Generally, for light microscopy or similar techniques, slides and covers of glass or another suitable transparent materials are used.

However, some tissue sections samples sticks strongly to microscopic slides and withstand several incubation and reaction steps. Other samples (e.g. lung) may be lost during staining methods.

A typical mounting process uses coated slide surfaces to mount tissue sections and increase their stickiness to the microscopic slide. As mentioned above, typical surface modifications are based on polylysin, collagen, gelatin, fibronectin, laminin, vitronectin, PEG, and materials that enhance the stickiness of tissue sections to the surface of an microscopic slide. Other methods claim the modifications of a glass surface by plasma treatment of the glass or a deposition of anorganic material like silanes or titanium dioxide (WO 2017/158238 A1). The disadvantage of coating is often their limited half life of mostly a couple of months.

Therefore, some researcher use also tissue treatments like an incubation with formaldehyde to increase adherance of tissue sections to glass slides. It is also well known that tissue section from taken paraffin embedded tissue blocks may change the stickiness to a surface. Formaldehyde that is also used for paraffin-embedded tissue samples for stabilization results in a non-directed cross-linking of biomolecules that may interfere with downstream analyses.

Downstream analyses of small quantities of analytes in biological and non-biological samples has become a routine practice in the clinical and analytical environment. Numerous analytical methods have been established for this purpose. Some of them use encoding techniques assigning a particular readable code to a specific first analyte which differs from a code assigned to a specific second analyte. Against this background, it is an object underlying the present disclosure to provide a method for mounting a biological sample on a surface by means of which the disadvantages of the prior art methods can be reduced or even avoided.

### SUMMARY OF THE DISCLOSURE

The present invention pertains to a method for mounting (or sticking/fixing) a biological sample on a surface like a microscopic slide.

For example, microscopy is a widely used method in biology and pathology. Some tissue sections samples sticks strongly to microscopic glass slides and withstand several incubation and reaction steps. Other samples (e.g. lung) may be lost during staining methods. The method of the present disclosure increases the success of analysing of those samples that are lost quite often during the process.

In a first aspect, embodiments of the disclosure pertains to a method of mounting a biological sample on a surface, which comprises:
i) applying a biological sample to a surface,
ii) contacting said biological sample on said surface with a reducing agent, followed by
iii) contacting said biological sample on said surface with an oxidizing agent.

In a preferred embodiment the disclosure pertains to a method of mounting (or sticking/fixing) a tissue on a slide and examination with a microscope, which comprises the steps of:
i) applying a tissue to the surface of a slide;
ii) contacting said tissue on said slide with an solution of a reducing agent;
iii) drying said slide to remove excess of the solvent;
iv) contacting said tissue on said slide with an aqueous solution of an oxidizing agent
   - microscopic examination of said tissue on said slide.

### DETAILED DESCRIPTION OF THE DISCLOSURE

The present invention relates to a method for mounting (or sticking) a biological sample on a surface like a microscopic slide. The present disclosure describes the usage of a reducing agent, in particular in combination with an oxidizing agent for increasing the success of mounting and than analyzng of biological samplse that are lost quite often during an analyzing process like a staining process for microscopy.

The method of the invention comprises the steps:
1) Bringing a biological sample into a contact with a surface
2) Incubating the biological sample at least by a reducing agent,
3) stopping the reducing reaction by at least an oxidizing reagent

Before step 1, the biomolecule containing sample may be incubated by a enzymatic, chemical, or physical treatment or a combination thereof to change features of the sample. Before step 1, the tissue sample may be cut by a microtome or cryotome, and tissue sections may be may be used for 1. Before step 1, cells, organoids, organoculture, or other biological material can be incubated in a medium or solution.

After step 1, a treatment may be required like deparaffinization, one or more enzymatic treatments, one or more chemical treatments, one or more physical treatments, or a mixture of them. The treatment of step 2 and 3 reduces the risk to loose the contact of the biological sample to the surface. Maybe that only step 2 is performed. The reducing reaction is able to reduce disulfid bridges within biomolecule containing samples. The reaction that stops the reducing reaction is able to oxidize reduced thiol groups. The biomolecule containing sample is immobilized during the treatment on flat surface for further analysis such as microscopy, spectrosocopy or other analyses.

Suprisingely, it is found by the inventors that for example a reaction that reduces disulfid-bounds in a first step and oxidize thiol groups in a second reaction results in a better stickiness of biomolecule containing material to a surface like glass or plastics.

As mentioned above, embodiments of the disclosure pertains to a method of mounting a biological sample on a surface, which comprises:
i) applying a biological sample to a surface,
ii) contacting said biological sample on said surface with a reducing agent, followed by
iii) contacting said biological sample on said surface with an oxidizing agent, optionally followed by
iv) staining/analyzing the mounted biological sample on said surface.

Furthermore, embodiments of the disclosure pertain to a method of mounting a biological sample on a surface, which comprises:
i) applying a biological sample to a surface,
ii) contacting said biological sample on said surface with a reducing agent, followed by
iii) contacting said biological sample on said surface with an oxidizing agent, optionally followed by
iv) staining/analyzing the mounted biological sample on said surface.

"Biological samples" means any material, including without limitation, blood, serum, fluid and tissue biopsy samples, collected from study subjects and any tangible material directly or indirectly derived there from. A "biological sample" or "biological specimen" is used herein to refer for example to a tissue section or cell smear. Prior to mounting the biological sample/specimen may be subjected to a series of physical and chemical manipulations that include sectioning and staining. Such physical and chemical manipulations are known to those of skill in the art and, thus, they will not be described only briefly hereinbelow. Once subjected to the foregoing physical and chemical manipulations, the biological sample/specimen may be referred to as a "histochemical section" or "cytochemical smear." As such, "histochemical section" refers to a solid sample of biological tissue which has been frozen or chemically fixed and/or hardened by embedding in wax or plastic, sliced into a thin sheet, generally several microns thick, and attached to a surface like a microscope slide. Moreover, "cytochemical smear" refers to a suspension of cells, such as blood cells, which should be fixed and attached to a surface like a microscope slide.

In particular, the biological sample is a biomolecule containing sample which may be described by its content of biomolecules that carry at least oxidized disulfid-bridges. The sample can be dead or alive. It is not necessary that all biomolecules within the sample comprise oxidized thiol-groups. The biomolecule containing sample may be an extract of biomolecules, cells (bacteria, archaebacteria, eukaryota etc.), tissue sample, tissue section, whole organ sample, whole organism, or multiple organisms. The sample may also comprise other material that do not include biomolecules. This also includes samples where extracts of biomolecules are added to the same.

"Biomolecules" are molecules that can be found in organisms and/or cells. This includes also molecules that were manipulated, digested, or modified by any type of biological, enzymatic, chemical, or physical treatment. This also includes extracted biomolecules added to a sample like a biological sample.

Therefore, in some advantageous embodiments, the biological sample comprises a biomolecule, a cell, a cell culture, a tissue section, an organoid and/or an organoculture, an organ, or an whole organism. In particular, the biological sample comprises a biomolecule that contains at least a disulfid-bridge, in particular an oxidized thiol-group.

In some advantageous embodiments, the biological sample comprises an extract of biomolecules, cells like prokaryotic, archaebacterial or eukaryotic cells, a tissue sample, a tissue section, a whole organ sample, a whole organism, or multiple organisms. In particular, the biological sample comprises blood, serum, fluid or a tissue like a biopsy sample.

In some embodiments, the biological sample biological sample is a tissue sample and the tissue is cut by a microtome or cryotome in tissue sections before applied to the surface. In particular, the biological sample comprises lung or skin tissue. In some embodiments, the biological sample is incubated in a medium or solution before applied to the surface.

In some further advantageous embodiments, the biological sample is treated by an enzymatic, chemical, or physical treatment or a combination thereof before applied to the surface. In an embodiement of the present disclosure, the biological sample comprises a Formalin-Fixed Paraffin-Embedded (FFPE) Tissue. In particular, in an embodiment of a method accoding to the present disclosure, the Formalin-Fixed Paraffin-Embedded (FFPE) Tissue is deparaffinized after applied to the surface, in particular by one or more enzymatic treatments, one or more chemical treatments, one or more physical treatments, or a mixture of them.

In some advantageous embodiments, the biological sample/specimen is fixed with a method according to the present disclosure, prior to mounting a coverslip on the the biological sample/specimen at the microscope slide.

"Fixing a biological sample/specimen", "sticking a biological sample/specimen" or "mounting a biological sample/specimen" to a surface like a microscope slide as used herein, refers to a sample of biological cells like a biological tissue which has been chemically treated to stabilize proteins and to strengthen cellular structures, particularly membranes, against disruption by solvent changes, temperature changes, mechanical stresses, and drying, Cells may be fixed in suspension or contained in a sample of tissue, such as might be obtained during autopsy, biopsy or surgery.

Moreover, cells or tissues to be examined may be embedded in warm, liquid paraffin wax. The wax, which both surrounds the tissue and infiltrates it, hardens on cooling, thereby supporting the tissue externally and internally. The resulting solid paraffin block is then trimmed to the appropriate shape before being sectioned. If ultrathin sections are required, the use of harder embedding and infiltrating materials, such as epoxy plastics, may be required. Such materials are initially in liquid form and are poured into small molds containing pieces of fixed tissues; on heating, the liquid undergoes polymerization to form a hard plastic.

The trimmed blocks containing the embedded samples are sectioned using a microtome. In this instrument, the block is sequentially swept over the blade of a knife that cuts the block into a series of thin sections. Such sections are then mounted on, i.e. , deposited on or attached to, a microscope slide and stained with dyes or chromogens of various colors that specifically attach to different molecular constituents of the cells. At this point, the coverslip can be mounted on the fixed tissue or cell specimen.

"Coverslip," as used herein, refers to a thin slip of glass, plastic or other transparent, polymeric material used for covering a biological specimen on a microscope slide that to be observed under a microscope. The coverslip should be of a sufficient length and width to cover the biological sample/specimen in its entirety. As sued herein "mounting a coverslip" refers to the mount of a coverslip onto a microscope slide having a biological sample/specimen thereon.

The surface on which the biological sample is mounted/fixed/sticked may be flat or round, smooth or rough, hydrophilic or lipophilic. Further, the surface may be made from various materials and may be based on glass, plastics, metal, quartz or any other type of material a surface can be made from. In an advategeous embodiment, the surface is on or is a microscopic slide. Furthermore, the surface may be modified or coated to introduce further functionalities.

A "reducing agent", or reductant, loses electrons and is oxidized in a chemical reaction. A reducing agent is typically in one of its lower possible oxidation states, and is known as the electron donor. A reducing agent is oxidized, because it loses electrons in the redox reaction. Examples of reducing agents include the earth metals, formic acid, and sulfite compounds. As used herein "Reducing Agent" includes in particular all agents and conditions that are able to reduce disulfid bounds. The reducing capability of reducing reagents may be measured as an electrode potential compared to the standard electrode potential. Reducing agents can change their reducing capability by certain reaction conditions that change temperature, pH, concentration, or other parameters (see Pettrucci, Ralph H. General Chemistry: Principles and Modern Applications. 9th. Upper Saddle River: Pearson Prentice Hall, 2007; Oxtoby, David W., H.P. Gillis, and Alan Campion. Principles of Modern Chemistry. 6th. Belmont: Thomson Brooks/Cole, 2008.). Therefore, the skilled artisan can change the agents and reactions parameters to reduce disulfid-bounds in biomolecules. Typical reducing agents that are used for biochemical reactions are Beta-Mercaptoethanol, Dithiothreitol (DTT), or Dithioerythrit. In addition many metal based compounds can be used as reducing reagents such as sodium borohydride (NaBH₄) or lithium aluminium hydride (LiAlH₄). In order to prequalify reducing agents for the purpose described here, a skilled atisan is able to choose reagents from the galavanic series.

In some advantageous embodiments, the reducing agent used in the methods according to the present disclosure is selected from the group consisting of beta-Mercaptoethanol, Dithiothreitol (DTT), Dithioerythrit, sodium borohydride (NaBH₄), lithium aluminium hydride (LiAlH₄), or another reducing reagent of the galavanic series.

An "oxidizing agent", or oxidant, gains electrons and is reduced in a chemical reaction. Also known as the electron acceptor, the oxidizing agent is normally in one of its higher possible oxidation states because it will gain electrons and be reduced. As used herein "oxidizing agent" includes in particularhalogens, potassium nitrate, and nitric acid, in particular all agents and conditions that are able to oxidize thiol groups. For example, the oxidizing capability of oxidizing reagents can be measured as an electrode potential compared to the standard electrode potential. Oxidizing agents can change their oxidizing capability by certain reaction conditions that change temperature, pH, concentration, or other parameters (see Pettrucci, Ralph H. General Chemistry: Principles and Modern Applications. 9th. Upper Saddle River: Pearson Prentice Hall, 2007; Oxtoby, David W., H.P. Gillis, and Alan Campion. Principles of Modern Chemistry. 6th. Belmont: Thomson Brooks/Cole, 2008.). From this view, a skilled arisan can change the agents and reactions parameters to oxidize thiol-groups in biomolecules. In particular, the biological sample/specimen, in particular the biomolecule containing sample is treated by the reducing and oxidizing reagent in a way so that the samples sticks more tightly to the surface. Typical oxidizing agents that are used for biochemical reactions peroxides such as hydrogen peroxide (H₂O₂), organic peroxides (e.g. tert-butyl hydroperoxide), or sodium perborate, potassium permangante, hypochlorites, and other oxdizing reagents which may be chosen by a researcher from the galavanic series.

In some advantageous embodiments, the oxidizing agent used in a method according to the present disclosure is selected from the group consisting of hydrogen peroxide (H₂O₂), an organic peroxide like tert-butyl hydroperoxide, sodium perborate, potassium permanganate, hypochlorite, or another oxidizing reagent of the galavanic series.

As mentioned above, the present disclosure pertains also to a method of mounting/sticking/fixing a tissue on a slide and examination with a microscope, which comprises:
- applying a tissue to the surface of a slide;
- contacting said tissue on said slide with an solution of a reducing agent;
- drying said slide to remove excess of the solvent;
- contacting said tissue on said slide with an aqueous solution of an oxidizing agent;
- microscopic examination of said tissue on said slide.

Furthermore, the present disclosure pertains also to kit for mounting/sticking/fixing a biological sample on a surface, which comprises a reducing agent and an oxidizing agent as decibed above.

As mentioned above, the mounted biological sample on said surface may be stained and/or analyzed. Staining is used to highlight important features of the biological sample like a tissue as well as to enhance e.g. the tissue contrast. Hematoxylin is a basic dye that is commonly used in this process and stains the nuclei giving it a bluish color while eosin (another stain dye used in histology) stains the cell's nucleus giving it a pinkish stain. However, there are other several staining techniques used for particular cells and components (Black, 2012). Staining is a commonly used medical process in the medical diagnosis of tumors in which a dye color is applied on the posterior and anterior border of the sample tissues to locate the diseased or tumorous cells or other pathological cells (Musumeci, 2014). In biological studies staining is used to mark cells and to flag nucleic acids, proteins or the gel electrophoresis to aid in the microscopic examination (Jackson & Blythe, 2013). In some cases, various multiple staining methods are used such as differential staining, double staining or the multiple staining (lyiola & Avwioro, 2011).

In some advategeous embodiements, the mounted/fixed biological sample on the surface is analyzed by spatial transcriptomics (or Spatial *omics) that means any kind of analysis where data from the sample are derived in a spatial manner from *in-situ* samples of tissues or whole organisms. The *in situ* sample may be a section of an organ or an organism. The *in-situ* sample may be not pretreated or pretreated in a way that is required for improving the result. Spatial*omics may included the detection of small molecules compunds of tissues or cells, proteins, DNA, and / or RNA. More preferentially, spatial*omics is restricted to proteins, DNA, and/or RNA. More preferentially, spatial*omics is restricted to DNA and / or RNA. More preferentially, spatial*omics is restricted to smFISH, in-situ sequencing or nucleic acid capturing methods. Even more preferentially, spatial*omics is restricted to smFISH. Even more preferentially, spatial*omics is restricted to any kind of sequential smFISH.

In particular, the spatial transcriptomics detecting comprises a multiplex method for detecting different analytes in the pathogen-comprising sample by sequential signal-encoding of said analytes as decibed in WO 2020/254519 A1, PCT/EP2021/066620 or PCT/EP2021/066668.

Further methods for analyzing/staining the mounted biological sample of the surface according to the present disclosure may be the analysis and detection of small quantities of analytes in biological samples which has become a routine practice in the clinical and analytical environment. Numerous analytical methods have been established for this purpose such as **FISH,** *in-situ* sequencing, methods that include captuiring of biomolecules or spectral methods (e.g., mass spectrometry). Some of them use encoding techniques assigning a particular readable code to a specific first analyte which differs from a code assigned to a specific second analyte.

One of the prior art techniques in this field is the so-called 'single molecule fluorescence *in-situ* hybridization' (smFISH) essentially developed to detect mRNA molecules in a sample. In Lubeck et al. (2014), Single-cell in situ RNA profiling by sequential hybridization, Nat. Methods 11(4), p. 360-361, the mRNAs of interest are detected via specific directly labeled probe sets. After one round of hybridization and detection, the set of mRNA specific probes is eluted from the mRNAs and the same set of probes with other (or the same) fluorescent labels is used in the next round of hybridization and imaging to generate gene specific color-code schemes over several rounds. The technology needs several differently tagged probe sets per transcript and needs to denature these probe sets after every detection round.

A further development of this technology does not use directly labeled probe sets. Instead, the oligonucleotides of the probe sets provide nucleic acid sequences that serve as initiator for hybridization chain reactions (HCR), a technology that enables signal amplification; see Shah et al. (2016), In situ transcription profiling of single cells reveals spatial organization of cells in the mouse hippocampus, Neuron 92(2), p. 342-357.

Another technique referred to as 'multiplexed error robust fluorescence in situ hybridization' (merFISH) is described by Chen et al. (2015), RNA imaging. Spatially resolved, highly multiplexed RNA profiling in single cells, Science 348(6233):aaa6090. There, the mRNAs of interest are detected via specific probe sets that provide additional sequence elements for the subsequent specific hybridization of fluorescently labeled oligonucleotides. Each probe set provides four different sequence elements out of a total of 16 sequence elements. After hybridization of the specific probe sets to the mRNAs of interest, the so-called readout hybridizations are performed. In each readout hybridization, one out of the 16 fluorescently labeled oligonucleotides complementary to one of the sequence elements is hybridized. All readout oligonucleotides use the same fluorescent color. After imaging, the fluorescent signals are destroyed via illumination and the next round of readout hybridization takes place without a denaturing step. As a result, a binary code is generated for each mRNA species. A unique signal signature of 4 signals in 16 rounds is created using only a single hybridization round for binding of specific probe sets to the mRNAs of interest, followed by 16 rounds of hybridization of readout oligonucleotides labeled by a single fluorescence color.

A further development of this technology improves the throughput by using two different fluorescent colors, eliminating the signals via disulfide cleavage between the readout-oligonucleotides and the fluorescent label and an alternative hybridization buffer; see Moffitt et al. (2016), High-throughput single-cell gene-expression profiling with multiplexed error-robust fluorescence in situ hybridization, Proc. Natl. Acad. Sci. U S A. 113(39), p. 11046-11051.

A technology referred to as 'intron seqFISH' is described in Shah et al. (2018), Dynamics and spatial genomics of the nascent transcriptome by intron seqFISH, Cell 117(2), p. 363-376. There, the mRNAs of interest are detected via specific probe sets that provide additional sequence elements for the subsequent specific hybridization of fluorescently labeled oligonucleotides. Each probe set provides one out of 12 possible sequence elements (representing the 12 'pseudo colors' used) per color-coding round. Each color-coding round consists of four serial hybridizations. In each of these serial hybridizations, three readout probes, each labeled with a different fluorophore, are hybridized to the corresponding elements of the mRNA-specific probe sets. After imaging, the readout probes are stripped off by a 55% formamide buffer and the next hybridization follows. After 5 color-coding rounds with 4 serial hybridizations each, the color-codes are completed.

EP 0 611 828 discloses the use of a bridging element to recruit a signal generating element to probes that specifically bind to an analyte. A more specific statement describes the detection of nucleic acids via specific probes that recruit a bridging nucleic acid molecule. This bridging nucleic acids eventually recruit signal-generating nucleic acids. This document also describes the use of a bridging element with more than one binding site for the signal generating element for signal amplification like branched DNA.

Player et al. (2001), Single-copy gene detection using branched DNA (bDNA) in situ hybridization, J. Histochem. Cytochem. 49(5), p. 603-611, describe a method where the nucleic acids of interest are detected via specific probe sets providing an additional sequence element. In a second step, a preamplifier oligonucleotide is hybridized to this sequence element. This preamplifier oligonucleotide comprises multiple binding sites for amplifier oligonucleotides that are hybridized in a subsequent step. These amplifier oligonucleotides provide multiple sequence elements for the labeled oligonucleotides. This way a branched oligonucleotide tree is build up that leads to an amplification of the signal.

A further development of this method referred to as is described by Wang et al. (2012), RNAscope: a novel in situ RNA analysis platform for formalin-fixed, paraffin-embedded tissues, J. Mol. Diagn. 14(1), p.22-29, which uses another design of the mRNA-specific probes. Here two of the mRNA-specific oligonucleotides have to hybridize in close proximity to provide a sequence that can recruit the preamplifier oligonucleotide. This way the specificity of the method is increased by reducing the number of false positive signals.

Choi et al. (2010), Programmable in situ amplification for multiplexed imaging of mRNA expression, Nat. Biotechnol. 28(11), p. 1208-1212, disclose a method known as 'HCR-hybridization chain reaction'. The mRNAs of interest are detected via specific probe sets that provide an additional sequence element. The additional sequence element is an initiator sequence to start the hybridization chain reaction. Basically, the hybridization chain reaction is based on metastable oligonucleotide hairpins that self-assemble into polymers after a first hairpin is opened via the initiator sequence.

A further analyzing method according to the present disclosure is the probabilistic cell typing by *in situ* sequencing (pciSeq), an approach that leverages prior scRNA-seq classification to identify cell types using multiplexed in situ RNA detection (Qian, X., Harris, K.D., Hauling, T. et al. Probabilistic cell typing enables fine mapping of closely related cell types in situ. Nat Methods 17, 101-106 (2020); Lee, J., Daugharthy, E., Scheiman, J. et al. Fluorescent in situ sequencing (FISSEQ) of RNA for gene expression profiling in intact cells and tissues. Nat Protoc 10, 442-458 (2015)).

A further development of the technology uses so called split initiator probes that have to hybridize in close proximity to form the initiator sequence for HCR, similarly to the RNAscope technology, this reduces the number of false positive signals; see Choi et al. (2018), Third-generation in situ hybridization chain reaction: multiplexed, quantitative, sensitive, versatile, robust. Development 145(12).

Mateo et al. (2019), Visualizing DNA folding and RNA in embryos at single-cell resolution, Nature Vol, 568, p. 49ff., disclose a method called 'optical reconstruction of chromatin structure (ORCA). This method is intended to make the chromosome line visible.

EP 2 992 115 B1 describes a method of sequential single molecule hybridization and provides technologies for detecting and/or quantifying nucleic acids in cells, tissues, organs or organisms through sequential barcoding..

### Methods and Examples

### 1) Experiment: Strength of tissue fixation

Sixteen sections of PAXgene (QIAGEN) fixed tissues (mouse skin, intestine, lung, and kidney) were placed on two glass slides. Sticky Slides (ibidi) were sticked to the glass slide so that the sixteen chambers are formed that can be individually adressed with different reaction conditions. All wells were treated by a rehydration process using 100% isopropanol, 95% Ethanol, 70% Ethanol, and an equilibration buffer. Individual solutions with or without 10 mM DTT were applied to to the individual wells and were incubated overnight. For details and results see table 1 below.

**Table 1: Conditions and results**

| Slide | Chamber | Tissue | Preincubation using +/-10 mM DTT, 60 min at 37°; followed by an incubation over-night in salt solution without DTT | Incubation over-night in salt solution including ... | Result |
|---|---|---|---|---|---|
| 1 | 1 | Skin | - | no DTT | Loss of tissue sample |
| | 2 | Intestine | - | no DTT | No loss |
| | 3 | Lung | - | no DTT | Almost detached completely |
| | 4 | Kidney | - | no DTT | No loss |
| | 5 | Skin | - | + DTT | Tissue flutters |
| | 6 | Intestine | - | + DTT | No loss |
| | 7 | Lung | - | + DTT | Almost detached after hybridization |
| | 8 | Kidney | - | + DTT | No loss |
| 2 | 1 | Skin | + | no DTT | Tissue flutters |
| | 2 | Intestine | + | no DTT | No loss |
| | 3 | Lung | + | no DTT | No loss |
| | 4 | Kidney | + | no DTT | No loss |
| | 5 | Skin | + | + DTT | No loss |
| | 6 | Intestine | + | + DTT | No loss |
| | 7 | Lung | + | + DTT | No loss |
| | 8 | Kidney | + | + DTT | No loss |

As shown in table 1, by using a pretreatment by DTT, tissues are immobilized much better to the glass slide. Without the DTT pretreatment 1 of 8 tissue sections were lost completely and 3 of 8 are detached so that certain regions are out of focus during microscopy by fluttering. Thus, 4 of 8 samples are not fixed completely. If a DTT pretreatment is performed, none of the 8 samples is lost and only one of the samples flutters.

### 2) Experiment: Fixation of lung samples to glass slides (CyFaV74)

Eight lung samples (PAXgene fixed; Qiagen) were immobilized on glass slides. All samples are treated in the same way with regards to rehydration, hybridization, colorization and washing steps. Some of the samples are preincubated at 37°C for 30 min by solutions that includes various concentrations of DTT. After DTT incubation, the reaction was stopped by H₂O₂. For details and results see table 2 below.

**Table 2: Conditions and results**

| Slide | Chamber | Tissue | Preincubation in 4xSSC +/-DTT | Stop | Result |
|---|---|---|---|---|---|
| 1 | 1 | Lung | - | - | Loss of tissue sample |
| | 2 | Lung | 100 mM DTT | - | No loss |
| | 3 | Lung | 25 mM DTT | - | No loss |
| | 4 | Lung | 10 mM DTT | - | No loss |
| | 5 | Lung | - | 1% H₂O₂ | Loss of tissue sample |
| | 6 | Lung | 100 mM DTT | 1% H₂O₂ | No loss |
| | 7 | Lung | 25 mM DTT | 1% H₂O₂ | No loss |
| | 8 | Lung | 10 mM DTT | 1% H₂O₂ | No loss |

As shown in table 2, the lung samples without the treatment by DTT or a combined treatment by DTT and H₂O₂ were lost. All other samples that underwent a DTT or the combined DTT/H₂O₂ treatment could be analyzed. A treatment by H₂O₂ did not fixate the sample to the glass slide.

### 3) Experiment: Fixation of lung samples with/without DTT (CyFaV77)

Eight lung samples (PAXgene fixed; Qiagen) were treated by a solution that includes sucrose and various concentration of DTT (see table below). Afterwards the samples were cut and immobiluzed on glass slides. All samples are treated in the same way with regards to rehydration, hybridization, colorization and washing steps. Some of the samples are incubated at 37°C for 30 min by solutions that includes various concentrations of DTT. After the incubation, all samples were incubated in 1% H₂O₂. For details and results see table 3 below.

**Table 3: Conditions and results**

| Slide | Chamber | Tissue | Incubation of lung sample prior to cutting | Pretreatment | Stop | Result |
|---|---|---|---|---|---|---|
| 1 | 1 | Lung | 30% Sucrose | - | | Loss of tissue sample |
| | 2 | Lung | 30% Sucrose | 100 mM DTT | | Loss of tissue sample |
| | 3 | Lung | 30% Sucrose | 100 mM DTT | 1% H₂O₂ | No loss |
| | 4 | Lung | 30% Sucrose | 10 mM DTT | 1% H₂O₂ | No loss |
| | 5 | Lung | 30% Sucrose +DTT | - | | No loss |
| | 6 | Lung | 30% Sucrose +DTT | 100 mM DTT | | No loss |
| | 7 | Lung | 30% Sucrose +DTT | 100 mM DTT | 1% H₂O₂ | No loss |
| | 8 | Lung | 30% Sucrose +DTT | 10 mM DTT | 1% H₂O₂ | No loss |

As shown in table 3, the lung samples without DTT treatment were lost. Other samples that underwent a DTT or the combined DTT/H₂O₂ treatment could be analyzed with a higher propability.

### 4) Experiment: Is the sensitivity of smFISH experiments affected the treatment with DTT and H₂O₂ (AMV1435)

For this experiment liver samples were used to test in smFISH experiments adverse effects of a treatment by DTT and H₂O₂. Kidney and brain samples stick nicely to the surface without any treatment by DTT and H₂O₂. This sample was used to look for adverse effects by DTT/H₂O₂ treatment in FISH experiments. All samples are treated in the same way with regards to rehydration, hybridization, colorization and washing steps. In some chambers a combined DTT/H₂O₂ treatment was performed. Chamber 1 to 4 served as controls. For details and results see table 4 below.

**Table 4: Conditions and results**

| Slide | Chamber | Tissue | Preincubation 4xSSC/ DTT | Stop | Combined number of spot signals from channel 1 and 2 |
|---|---|---|---|---|---|
| 1 | 1 | Kidney | - | - | 6932 |
| | 2 | Kidney | 100 mM DTT | - | 8272 |
| | 3 | Kidney | 100 mM DTT | 1% H₂O₂ | 13165 |
| | 4 | Kidney | 10 mM DTT | 1% H₂O₂ | 13820 |
| | 5 | Brain | - | - | 13854 |
| | 6 | Brain | 100 mM DTT | - | 19730 |
| | 7 | Brain | 100 mM DTT | 1% H₂O₂ | 9670 |
| | 8 | Brain | 10 mM DTT | 1% H₂O₂ | 19130 |

As shown in table 4, the number of spot signals is not significantly affected by the treatment with DTT and H₂O₂. Minor variations in spot signals derived from biological variation within different sections and regions used for the assay.

### 5) Experiment: Is the sensitivity of smFISH experiments affected the treatment with DTT and H₂O₂ (AMV1435)

For this experiment liver samples were used to test in smFISH experiments adverse effects of a treatment by DTT and H₂O₂. Liver sample sticks nicely to the surface without any treatment by DTT and H₂O₂. This sample was used to look for adverse effects by DTT/H₂O₂ treatment in FISH experiments. All samples are treated in the same way with regards to rehydration, hybridization, colorization and washing steps. In some chambers a combined DTT/H₂O₂ treatment was performed. Chamber 1 to 4 served as controls. For details and results see table 5 below.

**Table 5: Conditions and results**

| Slide | Chamber | Tissue | Preincubation 4xSSC/ DTT | Stop | Combined number of spot signals from channel 1 and 2 |
|---|---|---|---|---|---|
| 1 | 1 | Liver | - | - | 19946 |
| | 2 | Liver | - | - | 24275 |
| | 3 | Liver | - | - | 5148 |
| | 4 | Liver | - | - | 5319 |
| | 5 | Liver | 10 mM DTT | 1% H₂O₂ | 22995 |
| | 6 | Liver | 10 mM DTT | 1% H₂O₂ | 26746 |
| | 7 | Liver | 10 mM DTT | 1% H₂O₂ | 5876 |
| | 8 | Liver | 10 mM DTT | 1% H₂O₂ | 6685 |

As shown in table 5, the number of spot signals is not significantly reduced by the treatment with DTT and H₂O₂.

## Claims

1. A method of mounting a biological sample on a surface, which comprises:
i) applying a biological sample to a surface,
ii) contacting said biological sample on said surface with a reducing agent, followed by
iii) contacting said biological sample on said surface with an oxidizing agent.

2. The method according to claim 1, wherein said reducing agent is selected from the group consisting of beta-Mercaptoethanol, Dithiothreitol (DTT), Dithioerythrit, sodium borohydride (NaBH₄), lithium aluminium hydride (LiAlH₄), or another reducing reagent of the galavanic series.

3. The method according to any one of claims 1 to 2, wherein said oxidizing agent is selected from the group consisting of hydrogen peroxide (H₂O₂), an organic peroxide like tert-butyl hydroperoxide, sodium perborate, potassium permanganate, hypochlorite, or another oxidizing reagent of the galavanic series.

4. A method according to any of claims 1-3, wherein the surface is a slide for examination with a microscope, the method comprising
- applying a tissue to the surface of the slide;
- contacting said tissue on said slide with a solution of a reducing agent;
- drying said slide to remove excess of the solvent;
- contacting said tissue on said slide with an aqueous solution of an oxidizing agent;
- microscopic examination of said tissue on said slide.

5. The method according to claim 4, wherein said slide comprises a surface of glass, plastics, metal and/or quartz.

6. The method according to any one of claims 4 to 5, wherein said surface is flat or round, smooth or rough, hydrophilic or lipophilic.

7. The method according to any one of claims 4 to 6, wherein said surface is modified or coated to comprise further functionalities.

8. The method according to any one of claims 4 to 7, wherein said tissue is treated by an enzymatic, chemical, or physical treatment or a combination thereof before applied to the surface.

9. The method according to any one of claims 4 to 8, wherein said tissue is cut by a microtome or cryotome in tissue sections before applied to the surface.

10. The method according to any one of claims 4 to 9, wherein said tissue comprises lung or skin tissue.

11. The method according to any one of claims 4 to 10, wherein said tissue comprises a biomolecule that contains at least an disulfid-bridge, in particular an oxidized thiol-group.

12. The method according to any one of claims 4to 11, wherein said tissue is incubated in a medium or solution before applied to the surface.

13. The method according to any one of claims 4 to 12, wherein said tissue is a Formalin-Fixed Paraffin-Embedded (FFPE) Tissue.

14. The method according to claim 13, wherein said Formalin-Fixed Paraffin-Embedded (FFPE) Tissue is deparaffinized after applied to the surface, in particular by one or more enzymatic treatments, one or more chemical treatments, one or more physical treatments, or a mixture of them.

15. The method according to any one of claims 4 to 14, wherein said reducing agent is selected from the group consisting of beta-Mercaptoethanol, Dithiothreitol (DTT), Dithioerythrit, sodium borohydride (NaBH₄), lithium aluminium hydride (LiAlH₄), or another reducing reagent of the galavanic series, and wherein said oxidizing agent is selected from the group consisting of hydrogen peroxide (H₂O₂), an organic peroxide like tert-butyl hydroperoxide, sodium perborate, potassium permanganate, hypochlorite, or another oxidizing reagent of the galavanic series.

## Patentansprüche

1. Verfahren zum Aufbringen einer biologischen Probe auf eine Oberfläche, das Folgendes umfasst:
i) Aufbringen einer biologischen Probe auf eine Oberfläche,
ii) Inkontaktbringen der biologischen Probe auf der Oberfläche mit einem Reduktionsmittel, gefolgt von
iii) Inkontaktbringen der biologischen Probe auf der Oberfläche mit einem Oxidationsmittel.

2. Verfahren nach Anspruch 1, wobei das Reduktionsmittel ausgewählt ist aus der Gruppe bestehend aus Beta-Mercaptoethanol, Dithiothreitol (DTT), Dithioerythrit, Natriumborhydrid (NaBH₄), Lithiumaluminiumhydrid (LiAIH₄) oder einem anderen reduzierenden Reagenz der galvanischen Reihe.

3. Verfahren nach einem der Ansprüche 1 bis 2, wobei das Oxidationsmittel ausgewählt ist aus der Gruppe bestehend aus Wasserstoffperoxid (H₂O₂), einem organischen Peroxid wie tert-Butylhydroperoxid, Natriumperborat, Kaliumpermanganat, Hypochlorit oder einem anderen oxidierenden Reagenz der galvanischen Reihe.

4. Verfahren nach einem der Ansprüche 1 bis 3, wobei die Oberfläche ein Objektträger für die Untersuchung mit einem Mikroskop ist, das Verfahren umfassend
- Aufbringen eines Gewebes auf die Oberfläche des Objektträgers;
- Inkontaktbringen des Gewebes auf dem Objektträger mit einer Lösung eines Reduktionsmittels;
- Trocknen des Objektträgers, um überschüssiges Lösungsmittel zu entfernen;
- Inkontaktbringen des Gewebes auf dem Objektträger mit einer wässrigen Lösung eines Oxidationsmittels;
- mikroskopische Untersuchung des Gewebes auf dem Objektträger.

5. Verfahren nach Anspruch 4, wobei der Objektträger eine Oberfläche aus Glas, Kunststoff, Metall und/oder Quarz umfasst.

6. Verfahren nach einem der Ansprüche 4 bis 5, wobei die Oberfläche flach oder rund, glatt oder rau, hydrophil oder lipophil ist.

7. Verfahren nach einem der Ansprüche 4 bis 6, wobei die Oberfläche modifiziert oder beschichtet ist, um weitere Funktionalitäten zu umfassen.

8. Verfahren nach einem der Ansprüche 4 bis 7, wobei das Gewebe vor dem Aufbringen auf die Oberfläche einer enzymatischen, chemischen oder physikalischen Behandlung oder einer Kombination davon unterzogen wird.

9. Verfahren nach einem der Ansprüche 4 bis 8, wobei das Gewebe vor dem Aufbringen auf die Oberfläche mit einem Mikrotom oder Kryotom in Gewebeschnitte geschnitten wird.

10. Verfahren nach einem der Ansprüche 4 bis 9, wobei das Gewebe Lungen- oder Hautgewebe umfasst.

11. Verfahren nach einem der Ansprüche 4 bis 10, wobei das Gewebe ein Biomolekül umfasst, das mindestens eine Disulfidbrücke, insbesondere eine oxidierte Thiolgruppe, enthält.

12. Verfahren nach einem der Ansprüche 4 bis 11, wobei das Gewebe vor dem Aufbringen auf die Oberfläche in einem Medium oder einer Lösung inkubiert wird.

13. Verfahren nach einem der Ansprüche 4 bis 12, wobei das Gewebe ein formalinfixiertes, in Paraffin eingebettetes (FFPE) Gewebe ist.

14. Verfahren nach Anspruch 13, wobei das formalinfixierte, in Paraffin eingebettete (FFPE) Gewebe nach dem Aufbringen auf die Oberfläche entparaffiniert wird, insbesondere durch eine oder mehrere enzymatische Behandlungen, eine oder mehrere chemische Behandlungen, eine oder mehrere physikalische Behandlungen oder eine Mischung davon.

15. Verfahren nach einem der Ansprüche 4 bis 14, wobei das Reduktionsmittel ausgewählt ist aus der Gruppe bestehend aus Beta-Mercaptoethanol, Dithiothreitol (DTT), Dithioerythrit, Natriumborhydrid (NaBH₄), Lithiumaluminiumhydrid (LiAlH₄) oder einem anderen reduzierenden Reagenz der galvanischen Reihe und wobei das Oxidationsmittel ausgewählt ist aus der Gruppe bestehend aus Wasserstoffperoxid (H₂O₂), einem organischen Peroxid wie tert-Butylhydroperoxid, Natriumperborat, Kaliumpermanganat, Hypochlorit oder einem anderen oxidierenden Reagenz der galvanischen Reihe.

## Revendications

1. Procédé de montage d'un échantillon biologique sur une surface, qui comprend :
i) l'application d'un échantillon biologique sur une surface,
ii) la mise en contact dudit échantillon biologique sur ladite surface avec un agent réducteur, suivie de
iii) la mise en contact dudit échantillon biologique sur ladite surface avec un agent oxydant.

2. Procédé selon la revendication 1, dans lequel ledit agent réducteur est choisi dans le groupe constitué par le bêta-mercaptoéthanol, le dithiothréitol (DTT), le dithioérythrit, le borohydrure de sodium (NaBH₄), l'hydrure de lithium et d'aluminium (LiAIH₄) ou un autre réactif réducteur de la série galavanique.

3. Procédé selon l'une quelconque des revendications 1 à 2, dans lequel ledit agent oxydant est choisi dans le groupe constitué par le peroxyde d'hydrogène (H₂O₂), un peroxyde organique comme l'hydroperoxyde de tert-butyle, le perborate de sodium, le permanganate de potassium, l'hypochlorite ou un autre réactif oxydant de la série galavanique.

4. Procédé selon l'une quelconque des revendications 1 à 3, dans lequel la surface est une lame destinée à être examinée au microscope, le procédé consistant à
- appliquer un tissu sur la surface de la lame ;
- mettre en contact ledit tissu sur ladite lame avec une solution d'agent réducteur ;
- sécher ladite lame pour éliminer l'excès de solvant ;
- mettre en contact ledit tissu sur ladite lame avec une solution aqueuse d'un agent oxydant ;
- effectuer un examen microscopique dudit tissu sur ladite lame.

5. Procédé selon la revendication 4, dans lequel ladite lame comprend une surface en verre, en plastique, en métal et/ou en quartz.

6. Procédé selon l'une quelconque des revendications 4 à 5, dans lequel ladite surface est plate ou ronde, lisse ou rugueuse, hydrophile ou lipophile.

7. Procédé selon l'une quelconque des revendications 4 à 6, dans lequel ladite surface est modifiée ou revêtue pour comprendre d'autres fonctionnalités.

8. Procédé selon l'une quelconque des revendications 4 à 7, dans lequel ledit tissu est traité par un traitement enzymatique, chimique ou physique ou une combinaison de ceux-ci avant d'être appliqué sur la surface.

9. Procédé selon l'une quelconque des revendications 4 à 8, dans lequel ledit tissu est coupé par un microtome ou un cryotome en sections de tissu avant d'être appliqué sur la surface.

10. Procédé selon l'une quelconque des revendications 4 à 9, dans lequel ledit tissu comprend du tissu pulmonaire ou cutané.

11. Procédé selon l'une quelconque des revendications 4 à 10, dans lequel ledit tissu comprend une biomolécule qui contient au moins un pont disulfide, notamment un groupe thiol oxydé.

12. Procédé selon l'une quelconque des revendications 4 à 11, dans lequel ledit tissu est incubé dans un milieu ou une solution avant d'être appliqué sur la surface.

13. Procédé selon l'une quelconque des revendications 4 à 12, dans lequel ledit tissu est un tissu fixé au formol, enrobé de paraffine (FFPE).

14. Procédé selon la revendication 13, dans lequel ledit tissu fixé au formol et enrobé de paraffine (FFPE) est déparaffiné après avoir été appliqué sur la surface, notamment par un ou plusieurs traitements enzymatiques, un ou plusieurs traitements chimiques, un ou plusieurs traitements physiques, ou un mélange de ceux-ci.

15. Procédé selon l'une quelconque des revendications 4 à 14, dans lequel ledit agent réducteur est choisi dans le groupe constitué du bêta-mercaptoéthanol, du dithiothréitol (DTT), du dithioérythrit, du borohydrure de sodium (NaBH₄), de l'hydrure de lithium et d'aluminium(LiAlH₄), ou d'un autre réactif réducteur de la série galavanique, et dans lequel ledit agent oxydant est choisi dans le groupe constitué par le peroxyde d'hydrogène (H₂O₂), un peroxyde organique comme l'hydroperoxyde de tert-butyle, le perborate de sodium, le permanganate de potassium, l'hypochlorite ou un autre réactif oxydant de la série galavanique.
